# EUROPÄISCHE PATENTSCHRIFT

(11) **EP 1 959 889 B1**
(45) Veröffentlichungstag und Bekanntmachung des Hinweises auf die Patenterteilung: **06.10.2010**
(21) Anmeldenummer: 06829598.9
(22) Anmeldetag: 14.12.2006
(51) Int. Cl.: A61F 13/02

(54) **WUNDBEHANDLUNGSVORRICHTUNG MIT ELASTISCH VERFORMBAREM UNTERDRUCKERZEUGUNGSELEMENT**
WOUND TREATMENT DEVICE WITH ELASTICALLY DEFORMABLE VACUUM-GENERATING ELEMENT
DISPOSITIF DE TRAITEMENT DES BLESSURES DOTE D'UN ELEMENT ELASTIQUEMENT DEFORMABLE QUI CRÉE UNE DEPRESSION

(30) Priorität: 14.12.2005 DE 202005019670 U
(43) Veröffentlichungstag der Anmeldung: 27.08.2008
(73) Patentinhaber: Riesinger, Birgit, 48346 Ostbevern (DE)
(72) Erfinder: Riesinger, Birgit, 48346 Ostbevern (DE)
(74) Vertreter: Storz, Ulrich
(86) Internationale Anmeldenummer: PCT/EP2006/012041
(87) Internationale Veröffentlichungsnummer: WO 2007/068477

(56) Entgegenhaltungen:
- DE-A1- 19 844 355
- DE-U1-202004 017 052
- FR-A- 1 163 907
- US-A1- 2004 064 132

## Beschreibung

Die Erfindung betrifft eine Wundbehandlungsvorrichtung mit wenigstens einem elastisch verformbaren, direkt per Hand betätigbaren Unterdruckerzeugungselement, welches an einem folienartigen, den jeweiligen Wundhohlraum abdeckenden Wundabdeckungselement angeordnet und mit diesem schlauchlos verbunden ist.

Eine Wundbehandlungsvorrichtung der eingangs genannten Art ist der DE 198 44 355 A1 zu entnehmen. Das in Fig. 2 dargestellte Unterdruckerzeugungselement ist direkt mit der Wundabdeckungsfolie verklebt. Das Unterdruckerzeugungselement ist ein glockenförmiger, mit einem vorkomprimierten Schwamm gefüllter Hohlkörper, welcher während des Saugvorgangs erst dann aufquillt, wenn eine zwischen dem Schwamm und der Wundfläche liegende, wasserlösliche Platte sich auflöst. Der vorkomprimierte Schwamm drückt auch gegen die ganze Innenfläche des Hohlkörpers. Die Kompression und anschließendes Expandieren des Schwammes in Richtung Wunde kann auch ohne wasserlösliche Platte erfolgen, indem der Schwamm erst beim Anlegen es Verbandes zusammengedrückt und in dem Hohlkörper untergebracht wird. Nachteilig in beiden Fällen ist, dass der zusammengedrückte Schwamm beim Expandieren gegen die empfindliche Wundfläche drückt.

Aufgabe der Erfindung ist, eine verbesserte, mit der Wundabdeckungsfolie integrierte Wundbehandlungsvorrichtung mit Unterdruckerzeugungselement zu konzipieren, bei der auf die vorkomprimierte Füllung verzichtet werden kann.

Diese Aufgabe ist durch eine gattungsgemäße Wundbehandlungsvorrichtung gelöst, bei der
- das Unterdruckerzeugungselement ein Hohlkörper ist, dessen Hohlraum im am Körper des Patienten angebrachten Zustand über eine am Wundabdeckungselement eingearbeitete Öffnung direkt mit dem Wundhohlraum in Kontakt steht,
- die Wundbehandlungsvorrichtung wenigstens einen die Wundsekrete aufzusaugenden Absorptionskörper aufweist, der im Gebrauch in den Wundhohlraum platziert ist, und welcher mit einer feinporigen, flüssigkeitspermeablen Hülle umgeben ist.

Es wird an eine neuartige Wundbehandlungsvorrichtung angestrebt, bei der das Wundexsudat durch den Absorptionskörper aufgesaugt wird, wobei die Aufsaugfunktion zugleich von einem vereinfachten, per Hand betätigbaren Unterdrucksystem unterstützt wird. Dabei kann der Absorptionskörper ein Flachgebilde sein, dessen Endvolumen im Laufe des Absorptionsvorgangs stark zunimmt, ohne einen bemerkenswerten Druck auf die Wundfläche zu üben. Soll jedoch ein Druck auf die Wunde ausgeübt werden, kann auf wenigstens einen zusätzlichen, umhüllten oder nicht umhüllten Absorptionskörper gegriffen werden, der direkt auf die Wundfläche, also unterhalb des besagten flachen Absorptionskörpers gelegt werden kann. Der zusätzliche Absorptionskörper kann auch die Funktion einer Auffangschicht für die groben, klumpenartigen Ausscheidungen übernehmen. Der Absorptionskörper kann trocken oder gering vorbefeuchtet auf die Wundfläche gelegt werden.

Der zusätzliche Absorptionskörper kann ein perforiertes Säckchen mit darin enthaltenen Absorberpartikeln, ein Formstück aus Schaumstoff bzw. Vlies, gegebenenfalls mit Superabsorberteilchen sein. In das Säckchen können superabsorbierende Schaumstoffperlen eingeschüttet sein.

Bei dem umhüllten Absorptionskörper kann sich um einen solchen handeln, der mit Superabsorbentien durchsetzt ist. Die Hülle kann Poren aufweisen, deren Größe die der superabsorbierenden Partikeln im Wesentlichen nicht überschreitet. Dadurch wird erreicht, dass die aufgesogenen Wundsekrete bis zum Entfernen des Absorptionskörpers aus der Wunde innerhalb der Hülle verbleiben und der Verbesserung des Wundraumklimas, d. h. der Aufrechterhaltung des feuchten Milieus beitragen. Das Wundexsudat muss nicht unbedingt über eine zusätzliche Leitung abgeführt werden, es sei denn, dass sich um einen Überschuss an Wundexsudat handelt.

Der Absorptionskörper kann aus unterschiedlichen medizinisch unbedenklichen Materialien gefertigt sein, wie offenzelliger Schaumstoff, Gel oder Textil. Vorzugsweise besteht er aus wenigstens einer Lage eines zellulosehaltigen, Superabsorberteilchen aufweisenden, vliesartigen Textilmaterials, das einfach zu verarbeiten und zu konfektionieren ist. Der Absorptionskörper kann aus Alginatfasern bestehen oder diese enthalten. Es wird ausdrücklich darauf hingewiesen, dass der (die) im Wundhohlraum bzw. im Hohlraum des Hohlkörpers untergebrachte (-en) Absorptionskörper nicht vorkomprimiert ist (sind).

Schließlich kann das zu absorbierende Formstück bzw. das Säckchen mit darin enthaltenen Absorberpartikeln direkt auf die Wunde gelegt werden, ohne den besagten umhüllten, flächenhaften Absorptionskörper einsetzen zu müssen, dieses Beispiel ist jedoch nicht Teil der vorliegenden Erfindung.

Um die Keime abzutöten, kann der umhüllte Absorptionskörper und/oder der zusätzliche, vlies-.oder schaumstoffartige Absorptionskörper oder ein direkt auf die Wunde auflegbares, antiadhäsives Folienelement mit silber- oder kupferhaltigen Substanzen versehen sein, beispielsweise in nanokristalliner Form. Als antiadhäsives Folienelement kann ein,perforiertes, sogenanntes Wunddistanzgitter eingesetzt werden, das zwischen der Wundoberfläche und dem Absorptionskörper anzuordnen ist. Es kommen auch zinkhaltige Substanzen in Frage, die den Wundheilungsprozess unterstützen können.

Weiterhin kann der Absorptionskörper Carboxymethylcellulose, natürliche oder synthetischer Hyaluronsäure, Honig und/oder dessen Derivate, Propolis und/oder pflanzliche, pharmazeutisch wirksame Extrakte, wie Aloe vera, enthalten.

Der zusammendrückbare Hohlkörper kann eine beliebige äußere Form aufweisen, unter Voraussetzung, dass er schlauchlos mit dem Wundabdeckungselement verbunden ist und an diesem stabil sitzt. Der Hohlkörper kann prismatisch, beispielsweise quaderförmig sein. Vorzugsweise ist der Hohlkörper in der Form eines elastisch verformbaren, beispielsweise elastomeren Umdrehungskörpers ausgeführt. Der hohle Umdrehungskörper kann sphärisch, zylindrisch oder kugelig sein, er kann aber auch die Form einer Birne oder Ovalwalze haben. Eine besonders vorteilhafte Ausführungsform des Hohlkörpers kann ein quaderförmiger oder etwa zylindrischer Balg darstellen, der sich im Wesentlichen nur in eine Richtung, etwa senkrecht zum verlegten Wundabdeckungselement deformieren lässt.

Vorzugsweise ist der Hohlkörper mit einem umlaufenden Flachkragen verbunden, welcher direkt oder über einen Polsterring mit dem Wundabdeckungselement verbunden sein kann. Der Polsterring kann einen flachen bis runden bzw. torusförmigen Querschnitt aufweisen. Die Aufgabe des Polsterringes ist, den Druck auf den Hohlkörper beim Andrücken per Hand auf die Haut des Patienten sanft zu übertragen und regelmäßig zu verteilen. Der Polsterring kann aus beliebigen verformbaren, insbesondere elastomeren Material, wie Gummi oder Kunststoff, hergestellt sein.

Der Hohlkörper kann auch einstückig mit dem Wundabdeckungselement ausgeführt sein. Dies kann insbesondere bei den kleineren Formaten des Wundabdeckungselementes der Fall sein. Eine einstückige Ausführungsform kann sich weiterhin auf ein Produkt beziehen, das sich aus dem Hohlkörper, dem Wundabdeckungselement und dem umhüllten Absorptionskörper zusammensetzt. Als "einstückige" wird hier eine einteilige Ausführung, beispielsweise ein Formstück bezeichnet.

Die Wundbehandlungsvorrichtung kann mit wenigstens einem am Wundabdeckungselement abnehmbar oder schwenkbar angeordneten Fenster versehen sein, an dem wiederum der besagte Hohlkörper sitzt. In diesem Fall weist das Wundabdeckungselement wenigstens eine Aussparung zur Aufnahme des Fensters auf.

An das Ventil kann ein Unterdruck-Indikator angeschlossen oder anschließbar sein, mit dem der Patient selbst bzw. der Arzt den Unterdruckwert ablesen und gegebenenfalls durch das Betätigen des Hohlkörpers oder des Ventils ändern kann. Der Unterdruck-Indikator kann Bestandteil einer externen Pumpe sein. Das Unterdruckerzeugungselement selbst, also der Hohlkörper kann die ergänzende Funktion eines Unterdruck-Indikators übernehmen, wenn entsprechend skaliert wird. Der Unterdruck-Indikator kann beispielsweise ein skaliertes, unmittelbar an das Ventil angeschlossenes Glasrohr mit Kolben sein.

Mit der Wundbehandlungsvorrichtung gemäß Erfindung können u. a. folgende Wundarten behandelt werden:
- mechanische Wunden, wie Schnitt- und Stichverletzungen, Bisswunden, Schusswunden, Schürfwunden;
- iatrogene Wunden,
- thermische Wunden, wie Verbrennungen;
- chemische Wunden, wie Verätzungen mit Säuren oder Laugen;
- offene Wunden;
- perforierende Wunden und andere.

Nachfolgend sind noch einige, ausgewählte Verwendungsmöglichkeiten aufgelistet:
- als Verband zur Behandlung einer ödematösen oder entzündlich veränderten Wundregion;
- als Verband zur Behandlung einer mikrobiell belasteten Wundoberfläche, indem über die Saugkraft Keime oder Zelltrümmer mit eingeschlossen, dehydriert oder in anaerobe Gebiete des gequollenen Absorptionskörpers geführt werden;
- als Verband zur Entfernung inflammatorischer Zytokine, Metrixmetalloproteasen, TIMP's, degradiertem Fibronektin (zieht das Gewebe zusammen) oder anderen chronifizierenden Stoffen;
- als Verband zur Regulation der Luftfeuchtigkeit, da der Absorptionskörper die wässrigen Bestandteile über deren Dampfdruck wieder in die Luft entlässt;
- als Verband über einem primär applizierten Wunddistanzgitter oder einer Gaze als Sekundärverband ohne unmittelbaren flächenhaften Kontakt zur Wunde;
- als Verband unterhalb einer wasserdampfdurchlässigen Folie zur Erreichung eines atmungsaktiven Verbandes;
- als Verband bei einer Kompressionstherapie;
- als Verband bei einer die epitheliale Zellmigration und Granulation fordernden Madentherapie, bei der die Larven von Lucilia sericata eingesetzt werden; hierbei handelt es sich insbesondere um akute und chronische Wundinfektionen. Statt Larven kann eine durch die Maden abgesonderte Substanz, nämlich der Madenspeichel eingesetzt werden.

Die Vorteile der Erfindung bestehen insbesondere darin, dass:
- durch den Einsatz von umhülltem Absorptionskörper das feuchte Milieu innerhalb des Wundhohlraums aufrechterhalten werden kann,
- der Absorptionsvorgang durch die Luftevakuierung unterstützt werden kann,
- die Luftevakuierung durch den Patienten durchgeführt werden kann; hierbei reicht es, einen Druck per Hand oder Finger auf den Balg oder die Kalotte des Hohlkörpers auszuüben;
- der Absorptionskörper als Speicher für das Wundexsudat dienen kann; hierbei müssen die Flüssigkeiten nicht unbedingt aus dem Wundgebiet ausgeleitet werden; sie können vielmehr wundnah gebunden gesammelt werden;
- die Zeit und die Kosten der Wundheilung verringert werden können.

Ausführungsbeispiele der Erfindung sind anhand der Zeichnung näher erläutert. Die Figuren zeigen:
- Fig. 1: eine an die Haut des Patienten angeklebte Wundbehandlungsvorrichtung mit einem glo- ckenförmigen Hohlköper, in einer schemati- schen Darstellung;
- Fig. 2: die Wundbehandlungsvorrichtung gemäß Fig. 1 in einer perspektivischen Ansicht;
- Fig. 3: die die Wundbehandlungsvorrichtung gemäß Fig. 1 mit einer in ihrem Scheitelbereich angeordneten Verdickung, in einer schema- tischen Darstellung;
- Fig. 4: die die Wundbehandlungsvorrichtung gemäß Fig. 1 im Einsatz;
- Fig. 5: die die Wundbehandlungsvorrichtung gemäß Fig. 1 in einer Draufsicht auf das Wund- abdeckungselement;
- Fig. 6: die Wundbehandlungsvorrichtung gemäß Fig. 1, mit einem Polsterring, in einer schema- tischen Darstellung;
- Fig. 7: den Polsterring in Draufsicht-auf seine Flachseite;
- Fig. 8: einen Schnitt A-A gemäß Fig. 7;
- Fig. 9a: eine zweite Ausführungsform des glocken- förmigen Hohlkörpers, mit nach Innen zei- genden Vorsprüngen, in einer schematischen Ansicht;
- Fig. 9b: den Hohlkörper gemäß Fig. 9a mit aufge- quollenem Absorptionskörper;
- Figuren 10a bis 10c: eine weitere, einstückige Ausführungsform der Wundbehandlungsvorrichtung, ebenso in einer schematischen Darstellung;
- Figuren 11a bis 12: eine vierte, balgartige Ausführungsform der Wundbehandlungsvorrichtung, in einer schematischen Darstellung;
- Fig.13: eine Wundbehandlungsvorrichtung mit einem quaderförmigen, balgartigen Hohlkörper, in einer perspektivischen Ansicht;
- Fig. 14: die Wundbehandlungsvorrichtung gemäß Fig. 11, mit einem Polsterring, in einer sche- matischen Darstellung;
- Fig. 15: eine Wundbehandlungsvorrichtung mit einem ballartigen Hohlkörper, in einer schemati- schen Darstellung; und
- Fig. 16: einen Hohlkörper mit Bodenplattenelement, ebenso in einer schematischen Darstellung.

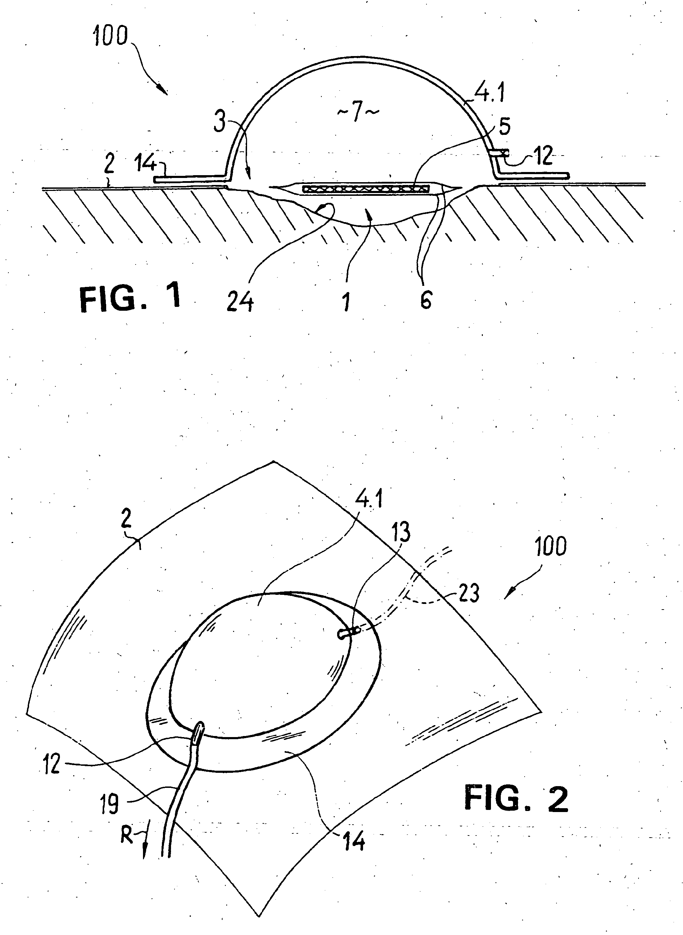

In Figuren 1 und 2 ist eine erste Ausführungsform (Bezugszahl 100) der Wundbehandlungsvorrichtung dargestellt, bestehend aus einem folienartigen Wundabdeckungselement . 2, einem glockenförmigen Hohlkörper 4.1 und einem Absorptionskörper 5. Der Hohlkörper 4.1 ist als Formstück aus Polyethylen im Tiefziehverfahren hergestellt. Die Wandungsdicke des transluzenten Hohlkörpers beträgt 0,8 mm. Das sphärische Formstück geht in einen umlaufenden Flachkragen 14 über, welcher auf das Wundabdeckungselement 2 vermittels eines medizinisch unbedenklichen Klebstoffes aufgeklebt ist. Die Wandung des Hohlkörpers begrenzt einen Hohlraum 7, der über eine am Wundabdeckungselement 2 eingebrachte Öffnung 3 direkt mit einem Wundhohlraum 1 in Kontakt steht. Der Wundhohlraum 1 ist durch eine mit 24 bezeichnete Wundfläche und das Wundabdeckungselement 2 definiert.

Der Hohlkörper 4.1 ist mit einem Einwegventil 12 versehen, das den Durchfluss von Luft und - nach Bedarf - von überschüssigen Wundsekreten, falls der Hohlkörper über eine zusätzlich vorgesehene Leitung 19 an eine entsprechende mechanische oder elektrische Ansaugvorrichtung (nicht dargestellt) angeschlossen ist, in einer Richtung (Pfeil R) erlaubt. Der Rückfluss von außen her ist also ausgeschlossen. Trotzdem kann ein weiteres Einwegventil 13 (vgl. Fig. 2) vorgesehen sein, mit dem sich der Druck innerhalb des Hohlkörpers und damit im Wundhohlraum 1 regulieren lässt. Über eine gestrichelt dargestellte Leitung 23 können Medikamente dosiert werden.

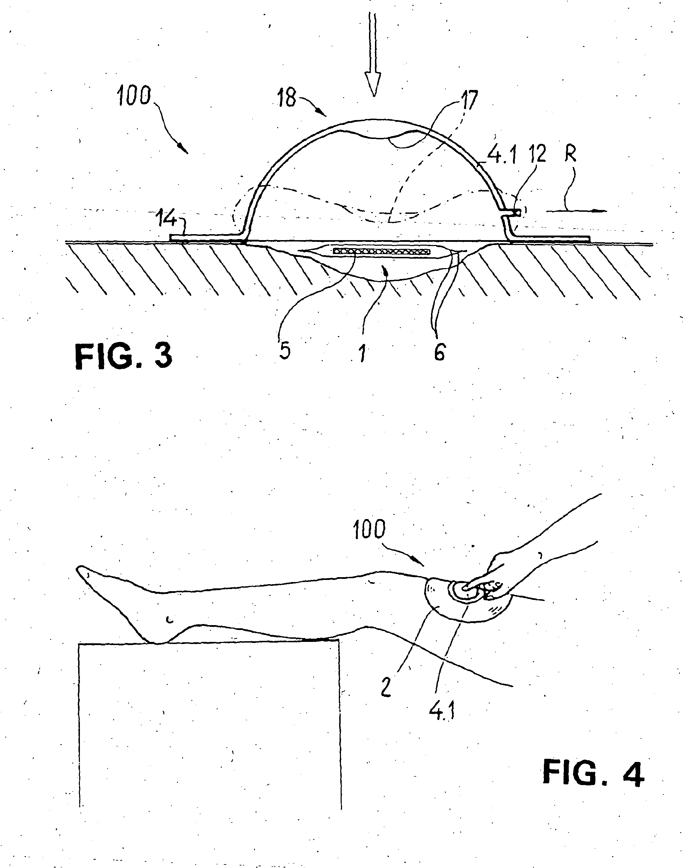

Die Figuren 1, 3 und 4 zeigen die Wundbehandlungsvorrichtung 100 im Einsatz. Am folienartigen Wundabdeckungselement 2 wird zuerst die Öffnung 3 der Wundgröße entsprechend ausgeschnitten und das Wundabdeckungselement 2 an die Haut des Patienten aufgeklebt. Der Absorptionskörper 5 wird in den Wundhohlraum 1 unterhalb des Wundabdeckungselementes 2 flach gelegt und erst dann wird der Hohlkörper 4.1 mit seinem Flachkragen 14 eingesetzt. Den Zustand zeigen die Figuren 1 und 3. Der Absorptionskörper 5 ist von einer perforierten Hülle 6 umgeben, deren Ausmaße (Breite und Länge, bzw. Durchmesser, wenn der Absorptionskörper rund ist) wesentlich größer als die des Absorptionskörpers sind. In vorliegendem Fall ist der Absorptionskörper 5 in Draufsicht auf seine Flachseite etwa 5,5 cm x 5,5 cm und die Hülle 6 etwa 7,0 cm x 7,0 cm groß.

Gemäß Fig. 3 weist der Hohlkörper 4.1 eine in Bereich seines Scheitels 18 liegende Verdickung 17 auf, mit der die Verformung des Hohlkörpers beim Andrücken per Hand, wie es die Fig. 4 zeigt, erleichtert werden kann.

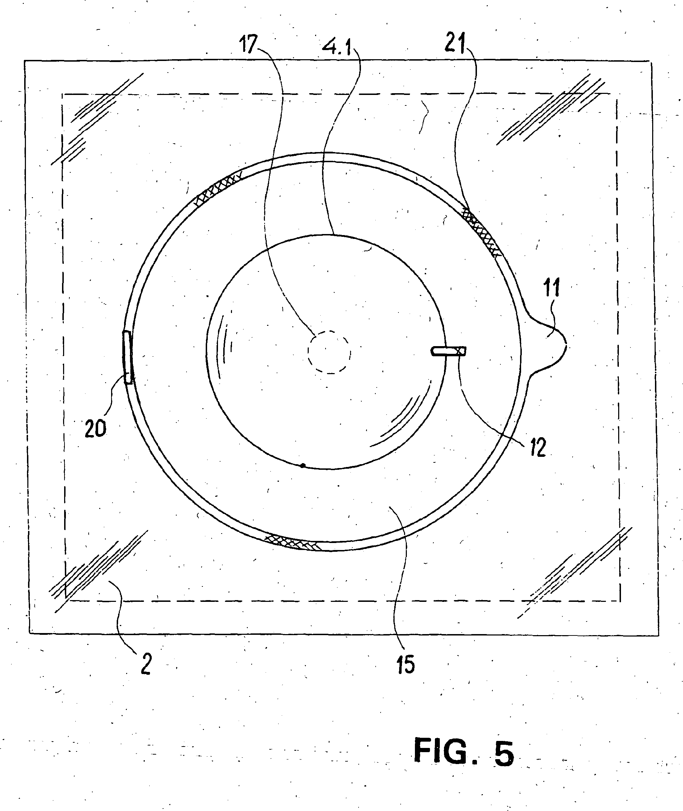

Die Ausführung gemäß Fig. 5 sieht vor, den Hohlkörper 4.1 an einem runden, über ein Folienscharnier 20 schwenkbaren Fenster 15 anzuordnen. Das Fenster 15 ist an seiner Unterseite peripher mit einem Release-Kleber 21 beschichtet, so dass es nach Bedarf, beispielsweise zwecks Herausnehmens des aufgequollenen Absorptionskörpers geöffnet und wieder aufgeklebt werden kann. Eine Ziehlasche 11 erleichtert die Handhabung mit dem Fenster.

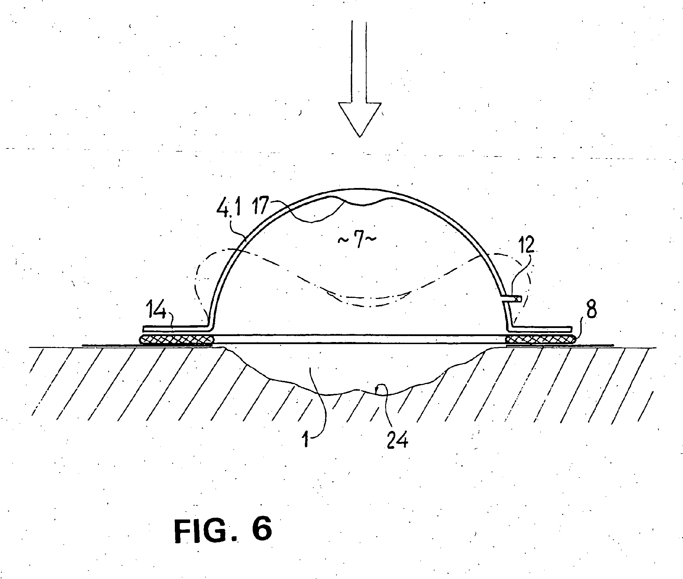

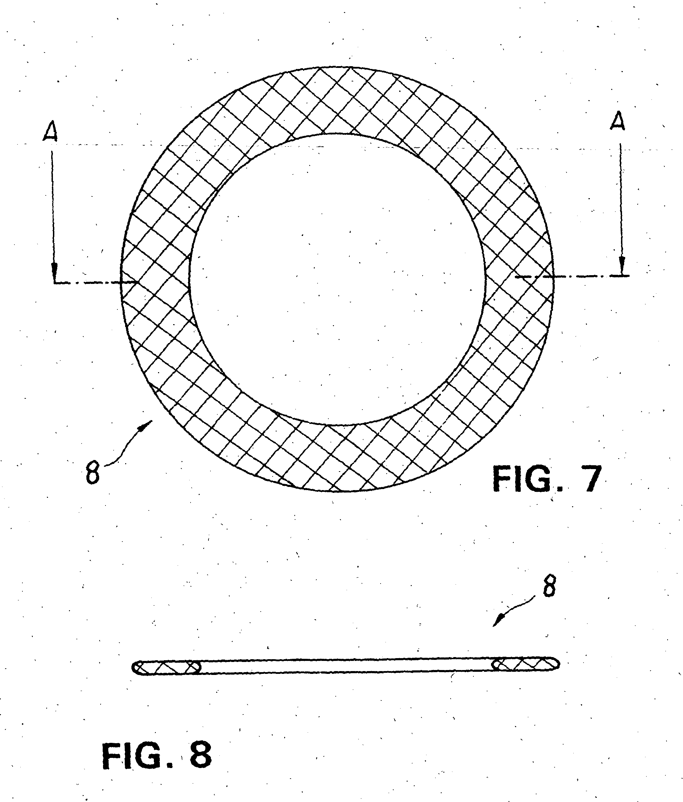

Der Hohlkörper 4.1 kann, wie es die Fig. 6 zeigt, sich mit seinem Flachkragen 14 an einem Polsterring 8 abstützen. Der Polsterring 8 (vgl. Figuren 7 und 8) ist aus einem elastomeren Material gefertigt, welches es erlaubt, die per Hand ausgeübten Andrückkräfte auf seine ganze Fläche zu verteilen. Vom Vorteil ist, dass der Polsterring 8 das effektive Volumen des Hohlraums 7 des Hohlkörpers 4.1 vergrößern kann.

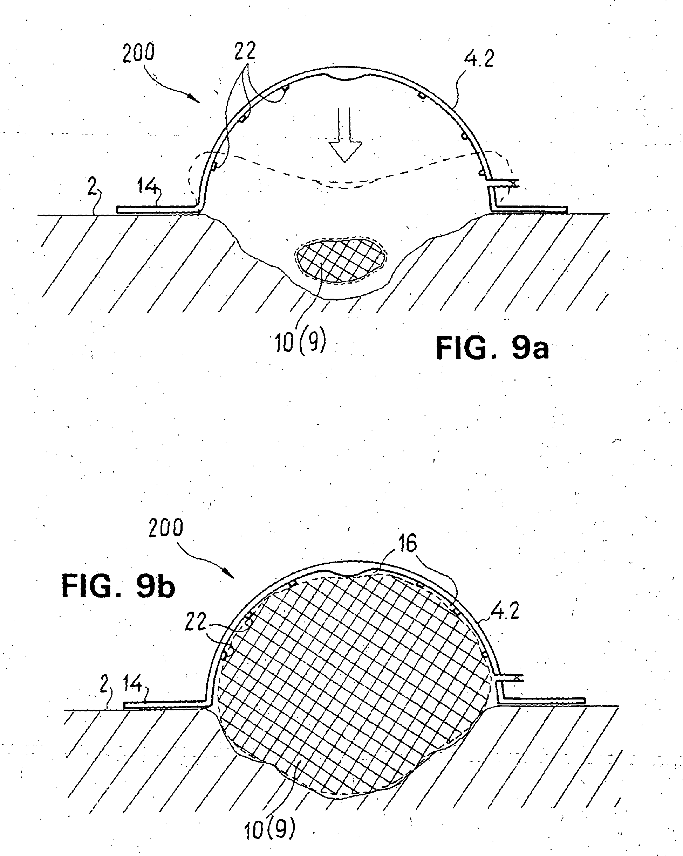

In den Figuren 9a und 9b ist eine ähnliche Ausführungsform (Bezugszeichen 200) der Wundbehandlungsvorrichtung gezeigt, bei der an Innenseite des Hohlkörpers 4.2 mehrere Abstandshalter 22 vorgesehen sind. In den Wundhohlraum 1 ist ein perforiertes Säckchen 10 mit einem darin enthaltenen, etwa linsenförmigen Absorptionskörper 9 gelegt. Quillt sich der Absorptionskörper 9 bis zu seinem maximalen Volumen (vgl. Fig.9b) auf, hindern die Abstandshalter 22 den Absorptionskörper 9 daran, den ganzen Hohlraum 7 wegzunehmen, da noch freie Räume 16 zwischen der Hülle 6 und der Innenfläche des Hohlkörpers 4.2 vorhanden sind, die es erlauben, die Dosierung von flüssigen Medikamenten über die Leitung 23 (vgl. Fig. 2) noch vor dem Herausnehmen des Absorptionskörpers 9 vorzunehmen.

Optional können die Absorptionskörper 5; 9 eine Menge an Nanopartikeln aus Silber, Kupfer oder Zink enthalten, die als antibakterielles Mittel nützlich sind.

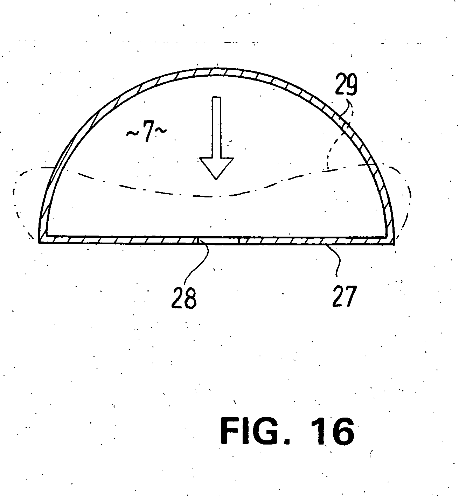

Die Fig. 16 zeigt einen Hohlkörper, dessen kalottenförmiges, zusammendrückbares Teil 29 in ein plattenförmiges Bodenelement 28 übergeht, an dem eine mittig liegende, mit dem Wundhohlraum 1 (nicht dargestellt) zu kontaktierende Öffnung 27 angeordnet ist. Das Bodenelement 28 ermöglicht eine gleichmäßige Druckverteilung beim Ausüben von Druckkraft mit der Hand.

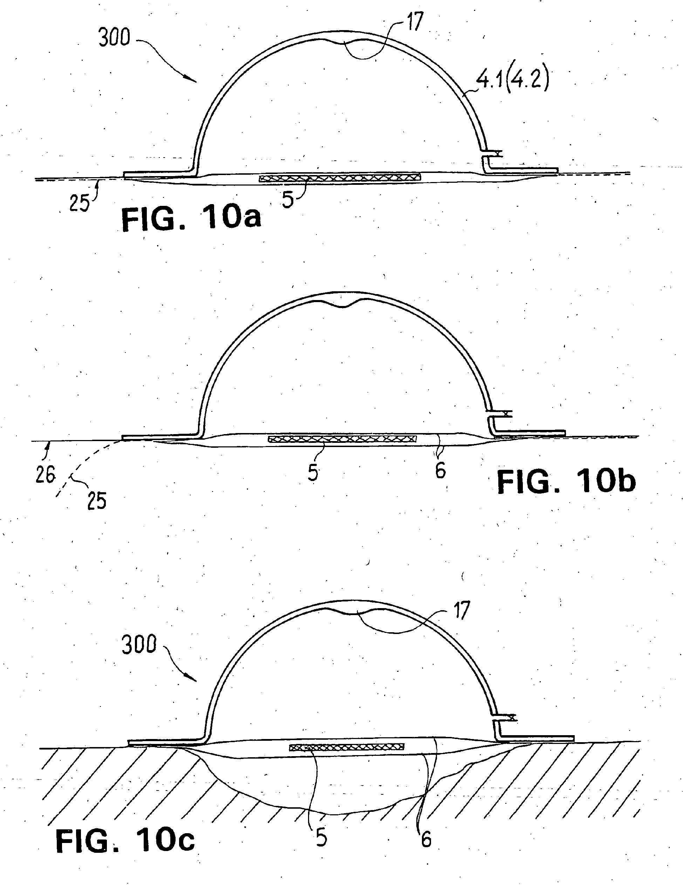

Die Figuren 10 a, 10b und 10c zeigen eine einstückige Wundbehandlungsvorrichtung 300, bestehend aus einem Hohlkörper 4.1 bzw. 4.2 mit einem Folienabschnitt des Wundabdeckungselementes, einem mit diesem verklebten Absorptionskörper 5 und einer abziehbaren, peripher angeordneten, ringförmigen Schutzfolie 25. Der Kragen 14 des Hohlkörpers 4.1 ist mit dem Wundabdeckungselement fest verklebt oder verschweißt. Vor dem Auflegen des Wundabdeckungselementes wird die Schutzfolie 25 abgezogen, so dass eine an der Unterseite des Wundabdeckungselementes befindliche Klebeschicht 26 freigelegt ist (vgl. Fig. 10b) und die Vorrichtung um die Wunde herum auf die Haut des Patienten aufgeklebt werden kann (vgl. Fig. 10c). Die Wundbehandlungsvorrichtung 300 ist als vorgefertigtes Einwegprodukt gedacht, das in unterschiedlichen Größen herstellbar ist.

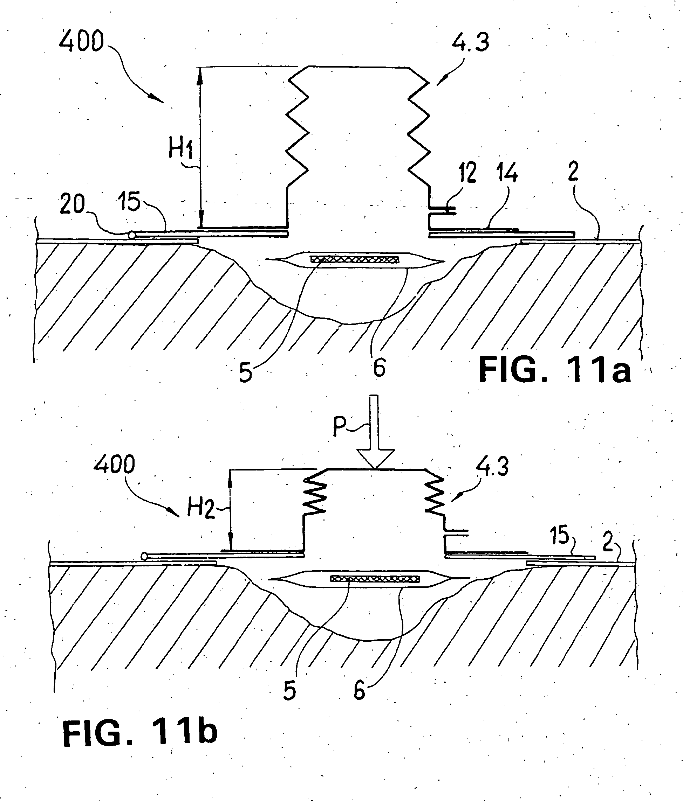

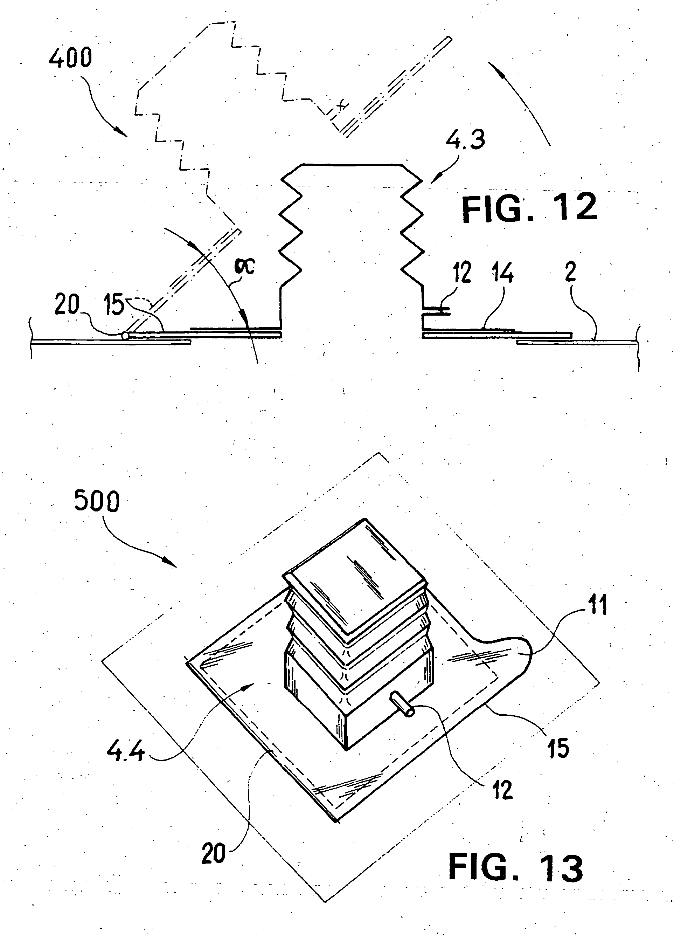

Die Figuren 11a, 11b und 12 zeigen eine Wundbehandlungsvorrichtung 400, die der in Fig. 5 dargestellten im Prinzip ähnlich ist, mit dem Unterschied, dass ihr Hohlkörper 4.3 die Form eines zylindrischen Balges hat, welcher im Wesentlichen nur in eine Richtung, die einer mit P bezeichneten Andrückkraft (vgl. Fig. 11b) entspricht, verformbar ist. Der Balg lässt sich sehr einfach mit der Hand oder mit dem Finger zusammendrücken. Die Fig. 12 zeigt wiederum den balgförmigen Hohlkörper 4.3 in zwei Stellungen. Der Hohlkörper 4.3 kann über das Folienscharnier 20 um einen sehr weiten Winkel α verschwenkt werden. Bei der Ausführungsform gemäß Fig. 13 handelt sich um eine Wundbehandlungsvorrichtung 500, die ein folienartiges, rechteckiges Fenster 15 hat, an dem ein quaderförmiger Hohlkörper 4.4 angeordnet ist. Das Konstruktionsprinzip der Wundbehandlungsvorrichtung 500 ist dem der Wundbehandlungsvorrichtung 400 gleich.

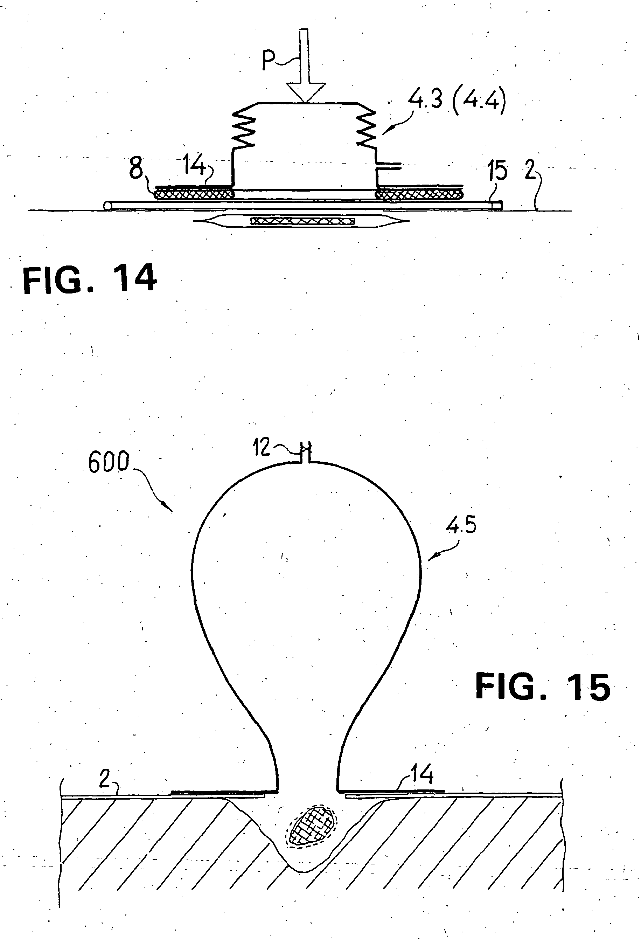

Wie die Fig. 14 zeigt, können sich die beiden Hohlkörper 4.3; 4.4 mit ihrem Flachkragen 14 ebenfalls am Polsterring 8 abstützen. Vorteilhaft ist, den Balg des Hohlkörpers so zu gestalten, dass sein sich aus dem Flachkragen 14 fortsetzender Teil kleinere Ausmaße (Durchmesser oder Breite) als die des zusammendrückbaren Teils hat. Diese Konfiguration ermöglicht, die Finger der Hand unter das zusammendrückbare, obere Teil hinein zu schieben und den Balg mit dem Daumen zu drücken, ohne den Druck auf das Wundabdeckungselement 2 ausüben zu müssen.

Schließlich ist in Fig. 15 eine Wundbehandlungsvorrichtung 600 dargestellt, derer mit dem Flachkragen 14 versehener Hohlkörper 4.5 etwa birnenförmig ist. Das Einwegventil 12 ist im Scheitelbereich zu sehen.

Alle beschriebenen Wundbehandlungsvorrichtungen 100 bis 600 bzw. deren Teile sind steril verpackt.

### Bezugszeichenliste:

- 1: Wundhohlraum
- 2: Wundabdeckungselement
- 3: Öffnung
- 4.1 bis 4.5: Hohlkörper
- 5: Absorptionskörper
- 6: Hülle
- 7: Hohlraum
- 8: Polsterring
- 9: Absorptionskörper
- 10: Säckchen
- 11: Ziehlasche
- 12; 13: Einwegventil
- 14: Flachkragen
- 15: Fenster
- 16: Raum
- 17: Verdickung
- 18: Scheitel
- 19: Leitung (zusätzlich)
- 20: Scharnier
- 21: Release-Kleber
- 22: Abstandshalter
- 23: Einwegventil
- 24: Wundfläche
- 25: Schutzfolie
- 26: Klebeschicht
- 27: Bodenelement
- 28: Öffnung
- 29: Teil

- α: Winkel
- P: Andrückkraft
- R: Richtung
- 100; 200; 300: Wundbehandlungsvorrichtung
- 400; 500; 600:

## Patentansprüche

1. Wundbehandlungsvorrichtung (100; 200; 300; 400; 500; 600) mit wenigstens einem elastisch verformbaren, direkt per Hand betätigbaren Unterdruckerzeugungselement, welches an einem folienartigen, den jeweiligen Wundhohlraum (1) abdeckenden Wundabdeckungselement (2) angeordnet und mit diesem schlauchlos verbunden ist,
**dadurch gekennzeichnet, dass**
das Unterdruckerzeugungselement ein Hohlkörper (4.1; 4.2; 4.3; 4.4; 4.5) ist, dessen Hohlraum (7) im am Körper des Patienten angebrachten Zustand über eine am Wundabdeckungselement (2) eingearbeitete Öffnung (3) direkt mit dem Wundhohlraum (1) in Kontakt steht, und dass die Wundbehandlungsvorrichtung wenigstens einen die Wundsekrete aufzusaugenden Absorptionskörper (5; 9) aufweist, der im Gebrauch in den Wundhohlraum (1) platziert ist und welcher mit einer feinporigen, flüssigkeitspermeablen Hülle (6) umgeben ist.

2. Wundbehandlungsvorrichtung nach Anspruch 1 , **dadurch gekennzeichnet, dass** der Hohlkörper (4.1; 4.2; 4.3) glocken- bzw. schalenförmig ist.

3. Wundbehandlungsvorrichtung nach Anspruch 1, **dadurch gekennzeichnet, dass** der Hohlkörper (4.4) balgförmig ist.

4. Wundbehandlungsvorrichtung nach Anspruch 1, **dadurch gekennzeichnet, dass** der Hohlkörper (4.5) die Form eines Balls bzw. einer Birne hat.

5. Wundbehandlungsvorrichtung nach Anspruch 1, **dadurch gekennzeichnet, dass** der Hohlkörper (4.1; 4.2; 4.3; 4.4; 4.5) einen mit dem Wundabdeckungselement (2) verbundenen Kragen (14) aufweist.

6. Wundbehandlungsvornchtung nach Anspruch 1, **dadurch gekennzeichnet, dass** der Hohlkörper (4.1; 4.2; 4.3; 4.4; 4.5) in einem Stück mit dem Wundabdeckungselement (2) ausgeführt ist.

7. Wundbehandlungsvorrichtung nach Anspruch 1, **dadurch gekennzeichnet, dass** sich der Hohlkörper (4.1; 4.2; 4.3; 4.4; 4.5) an einem Polsterring (8) abstützt.

8. Wundbehandlungsvorrichtung nach Anspruch 1, **dadurch gekennzeichnet, dass** die Wundbehandlungsvorrichtung mit wenigstens einem Ventil (12; 13) versehen ist.

9. Wundbehandlungsvorrichtung nach Anspruch 8, **dadurch gekennzeichnet, dass** das Ventil (12; 13) am Hohlkörper (4.1; 4.2; 4.3; 4.4; 4.5) angeordnet ist.

10. Wundbehandlungsvorrichtung nach Anspruch 1, **dadurch gekennzeichnet, dass** der Absorptionskörper (9) im nicht aufgequollenen Zustand eine von der flächenhaften abweichende Form, wie Linsenform, aufweist.

11. Wundbehandlungsvorrichtung nach Anspruch 1, **dadurch gekennzeichnet, dass** der Absorptionskörper (5; 9) mit superabsorbierenden Partikeln durchsetzt ist.

12. Wundbehandlungsvorrichtung nach Anspruch 1, **dadurch gekennzeichnet, dass** die Hülle (6) Poren aufweist, deren Grösse die der superabsorbierenden Partikeln im Wesentlichen nicht überschreitet.

13. Wundbehandlungsvorrichtung nach einem der vorhergehenden Ansprüche, **dadurch gekennzeichnet, dass** der Absorptionskörper (5; 9) schwammartig ist.

14. Wundbehandlungsvorrichtung nach einem der vorhergehenden Ansprüche, **dadurch gekennzeichnet, dass**
die Öffnung (3) des Wundabdeckungselementes (2) ein anbnehmbar oder schwenkbar angeordnetes Fenster (15) aufweist,
und dass der Hohlkörper (4.1; 4.2; 4.3; 4.4; 4.5) am Fenster (15) angeordnet ist.

15. Wundbehandlungsvornchtung nach einem der vorhergehenden Ansprüche, **dadurch gekennzeichnet, dass** der Hohlkörper (4.1; 4.2; 4.3; 4.4; 4.5) mit einem Bodenelement (27) versehen ist, das wiederum eine mittige Öffnung (28) aufweist.

16. Wundbehandlungsvorrichtung nach einem der vorhergehenden Ansprüche, **dadurch gekennzeichnet, dass** der Absorptionskörper (5; 9) Carboxymethylcellulose enthält.

## Claims

1. A wound treatment device (100; 200; 300; 400; 500; 600) having at least one elastically deformable vacuum producing element, which can be operated directly by hand, disposed on a film-like wound cover element (2) covering the respective wound cavity (1) and which is connected tubeless to the wound cover element (2),
wherein
the vacuum producing element is a hollow body (4.1; 4.2; 4.3; 4.4; 4.5) having a cavity (7) that is adapted to communicate with the wound cavity (1) via an opening (3) worked into the wound cover element (2) when the wound cover element is placed on the patient's body, and wherein the wound treatment device further comprises at least one wound secretions absorbing absorption body (5; 9) that is to be placed in the wound cavity during use and which is surrounded by a fine-pore, liquid-permeable envelope (6).

2. The wound treatment device according to claim 1, wherein the hollow body (4.1; 4.2; 4.3) is bell- or shellshaped.

3. The wound treatment device according to claim 1, wherein the hollow body (4.4) is in the shape of a bellows.

4. The wound treatment device according to claim 1, wherein the hollow body (4.5) has the shape of a ball or a pear.

5. The wound treatment device according to claim 1, wherein the hollow body (4.1; 4.2; 4.3; 4.4; 4.5) has a collar (14), which is connected to the wound cover element (2).

6. The wound treatment device according to claim 1, wherein the hollow body (4.1; 4.2; 4.3; 4.4; 4.5) is designed as a single piece with the wound cover element (2).

7. The wound treatment device according to claim 1, wherein the hollow body (4.1; 4.2; 4.3; 4.4; 4.5) is supported by a cushion ring (8).

8. The wound treatment device according to claim 1, wherein the wound treatment device is provided with at least one valve (12; 13).

9. The wound treatment device according to claim 9, wherein the valve (12; 13) is disposed at the hollow body (4.1; 4.2; 4.3; 4.4; 4.5).

10. The wound treatment device according to claim 1, wherein the absorption body (9) has a shape other than sheetlike such as lens-shaped in a nonswollen condition.

11. The wound treatment device according to claim 1, wherein the absorption body (5; 9) is interspersed with superabsorbing particles.

12. The wound treatment device according to claim 1, wherein the envelope (6) has pores whose size does not greatly exceed that of the superabsorbing particles.

13. The wound treatment device according to claim 1, wherein the absorption body (5; 9) is spongelike.

14. The wound treatment device according to anyone of the preceding claims, wherein the opening (3) of the wound cover element (2) comprises a window (15) disposed in such a way that window can be removed or swiveled and wherein the hollow body (4.1; 4.2; 4.3; 4.4; 4.5) is disposed at the window (15).

15. The wound treatment device according to anyone of the preceding claims, wherein the hollow body (4.1; 4.2; 4.3; 4.4; 4.5) is provided with a bottom element (27), which comprises a centered opening (28).

16. The wound treatment device according to anyone of the preceding claims, wherein the absorption body (5; 9) contains carboxymethylcellulose.

## Revendications

1. Dispositif de traitement de plaie (100 ; 200 ; 300 ; 400 ; 500 ; 600) avec au moins un élément producteur de dépression, pouvant être déformé de manière élastique et pouvant être actionné directement à la main, qui est placé sur un élément de recouvrement de plaie (2) du type film et recouvrant l'espace creux de plaie respectif (1) et qui est relié à cet élément de recouvrement de plaie sans tuyau,
**caractérisé en ce que** l'élément producteur de dépression est un corps creux (4.1 ; 4.2 ; 4.3 ; 4.4 ; 4.5) dont l'espace creux (7), lorsque le dispositif est appliqué sur le corps du patient, est en contact direct avec l'espace creux de plaie (1) par une ouverture (3) pratiquée dans l'élément de recouvrement de plaie (2)
et **en ce que** le dispositif de traitement de plaie comporte au moins un corps absorbant (5 ; 9) qui absorbe les sécrétions de la plaie, qui est placé lors de son utilisation dans l'espace creux de plaie (1) et qui est entouré d'une enveloppe (6) à pores fins et perméable au liquide.

2. Dispositif de traitement de plaie selon la revendication 1, **caractérisé en ce que** le corps creux (4.1 ; 4.2 ; 4.3) est en forme de cloche ou de coque.

3. Dispositif de traitement de plaie selon la revendication 1, **caractérisé en ce que** le corps creux (4.4) est en forme de soufflet.

4. Dispositif de traitement de plaie selon la revendication 1, **caractérisé en ce que** le corps creux (4.5) est en forme de balle ou de poire.

5. Dispositif de traitement de plaie selon la revendication 1, **caractérisé en ce que** le corps creux (4.1 ; 4.2 ; 4.3 ; 4.4 ; 4.5) comporte un col (14) relié à l'élément de recouvrement de plaie (2).

6. Dispositif de traitement de plaie selon la revendication 1, **caractérisé en ce que** le corps creux (4.1 ; 4.2 ; 4.3 ; 4.4 ; 4.5) est réalisé d'une seule pièce avec l'élément de recouvrement de plaie (2).

7. Dispositif de traitement de plaie selon la revendication 1, **caractérisé en ce que** le corps creux (4.1 ; 4.2 ; 4.3 ; 4.4 ; 4.5) repose sur un bourrelet annulaire (8).

8. Dispositif de traitement de plaie selon la revendication 1, **caractérisé en ce que** le dispositif de traitement de plaie est muni d'au moins une valve (12 ; 13).

9. Dispositif de traitement de plaie selon la revendication 8, **caractérisé en ce que** la valve (12 ; 13) est placée sur le corps creux (4.1 ; 4.2 ; 4.3 ; 4.4 ; 4.5).

10. Dispositif de traitement de plaie selon la revendication 1, **caractérisé en ce que** le corps absorbant (9) présente, lorsqu'il n'est pas gonflé, une forme différente d'une forme plane, par exemple une forme de lentille.

11. Dispositif de traitement de plaie selon la revendication 1, **caractérisé en ce que** le corps absorbant (5 ; 9) est chargé de particules superabsorbantes.

12. Dispositif de traitement de plaie selon la revendication 1, **caractérisé en ce que** l'enveloppe (6) comporte des pores dont la dimension ne dépasse globalement pas celle des particules superabsorbantes.

13. Dispositif de traitement de plaie selon l'une des revendications précédentes, **caractérisé en ce que** le corps absorbant (5 ; 9) est spongieux.

14. Dispositif de traitement de plaie selon l'une des revendications précédentes, **caractérisé en ce que** l'ouverture (3) de l'élément de recouvrement de plaie (3) comporte une fenêtre (15) agencée de manière à pouvoir être enlevée ou à pouvoir pivoter et **en ce que** le corps creux (4.1 ; 4.2 ; 4.3 ; 4.4 ; 4.5) est agencé sur la fenêtre (15).

15. Dispositif de traitement de plaie selon l'une des revendications précédentes, **caractérisé en ce que** le corps creux (4.1 ; 4.2 ; 4.3 ; 4.4 ; 4.5) est muni d'un fond (27) qui comporte lui-même une ouverture centrée (28).

16. Dispositif de traitement de plaie selon l'une des revendications précédentes, **caractérisé en ce que** le corps absorbant (5 ; 9) contient de la cellulose carboxyméthylique.
